# EUROPEAN PATENT SPECIFICATION

(11) **EP 0 391 218 B1**
(45) Date of publication and mention of the grant of the patent: **13.03.1996**
(21) Application number: 90105855.2
(22) Date of filing: 28.03.1990
(51) Int. Cl.: A61K 31/19, A61K 31/23, A61K 31/16, A61K 31/557, A61K 31/66, A61K 31/355, A61K 31/375, A61K 38/00, A61K 38/08, A61K 33/06, A61K 33/26

(54) **Agent for the atopy prophylaxis**
Mittel zur Atopieprophylaxe
Produit prophylactique pour l'atopie

(30) Priority: 04.04.1989 DE 3910761; 14.09.1989 DE 3930816
(43) Date of publication of application: 10.10.1990
(73) Proprietor: Beiersdorf Aktiengesellschaft, D-20245 Hamburg (DE)
(72) Inventor: Melnik, B., Dr. med, 4018 Langenfed (DE); Plewig, G., Prof. Dr. med., 4000 Düsseldorf 30 (DE)
(74) Representative: Werner, Hans-Karsten, Dr.

(56) References cited:
- EP-A- 0 068 854
- EP-A- 0 092 085
- EP-A- 0 115 419
- EP-A- 0 292 403
- BE-A- 897 806
- PATENT ABSTRACT OF JAPAN, vol. 11, no. 353 (C-457)[2800], 18 November 1987; & JP-A-62 126 933
- PATENT ABSTRACTS OF JAPAN, vol. 12, no. 37 (C-473)[2884], 4th February 1988; & JP-A-62 186 762
- BRITISXH JOURNAL OF DERMATOLOGY, 1987, vol. 117, no. 1, pp. 11-19; M. SCHALIN-KARRILA et al.: "Evening primrose oil in the treatment of atopic eczema: effect on clinical staturs, plasma phospholipid fatty acids and circulating blood prostaglandins"
- MEDICAL HXPOTHESES, 1979, vol. 5, no. 9, pp. 969-985; D.F. HORROBIN et al.: "The nuritional regulation of T lymphocyte function"
- MEDICAL HYPOTHESES, 1979, vol. 5, no. 8, pp. 849-858; D.F. HORROBIN et al.: "The regulation of prostaglandin et formation: a candidate for one of the fundamental mechanisms involved in the actions of vitamin C"
- HAUTARZT, vol. 40, no. 11, November 1989, pp. 685-692; B. MELNIK et al.: "Ein neues Konzept zur Ätiopathogenese und Prävention der Atopie"

## Description

The invention relates to the use of substances for preparing agents for atopy prophylaxis at a time when conditioning and thorough maturing of the cellular immune system has not yet been completed.

Atopy is generally understood to mean a familiarly occurring hypersensitivity to environmental substances of skin and mucosae, with an increase predisposition to develop hypersensitivity reactions of the immediately occurring type to substances originating from natural environment. It is supposed that the environmental substances induce the formation of antibodies of class IgE (immunoglobulin E). These IgE antibodies initiate the further allergic reactions. Then the atopy becomes manifest in the patient at the loci of contact with the so-called allergens (environment-derived substances such as pollen, spores, domestic dust etc.), for example
- in the eyes as an allergic conjunctivitis;
- at the nasal mucosa as an allergic rhinitis (hay fever);
- in the lungs as an allergic bronchial asthma;
- on the skin as chronic or chronic-relapsing dermatitis which is probably not IgE-mediated.

Atopy appears to have genetic reasons and is more or less markedly encountered with about 10 % of the population, with a recently increasing tendency of occurrence. Therefore, medical science for long has nurtured the desire for a long-lasting prevention of atopy and an enhancement of the quality of life.

It was already in the 1920's that it has been recognized that the nutrition in the early childhood in general is of crucial importance in preventive medicine. However, still in 1986 R.S. Zeiger et al., in "Journal of "Journal of Allergy and Clinical Immunology", 1986, Volume 78 (1 Part 2), pp. 224 to 238, stated that alone breast-feeding in combination with a delayed supply of solid nutrients is suitable to alleviate an atopic dermatitis and food allergies in the early childhood stage. Similar results have been obtained by U.M. Saarinen, et al. (in "The Lancet", July 28, 1979, pp. 163 to 166). The authors of this clinical study attempt to explain the effect of prolonged breast-feeding in terms of that due to the immunoglobulin A secrete in human milk the intestinal mucosa is blocked from the action of antigens.

From Chemical Abstracts, Vol. 98: 69 996v, it has been known that gamma-linolenic acid in the form of Evening Primrose Oil (Efamol) is capable of partially correcting the biochemical anomalities and the clinical status in atopy once already manifest.

From Chemical Abstracts, Vol. 100: 80 453w, it has been known that the increased capillary permeability in skin as caused by intravenous injection of chemical mediators such as histamine, serotonin and bradykinin, is suppressed by the subcutaneous injection of Prostaglandin E₁. The respective investigations were carried out with rats to demonstrate the *in vivo* effect of prostaglandins on the capillary permeability in the immediate type of allergic reactions.

Chemical Abstracts, Vol. 109: 209 935x, relates to a food composition containing omega-6-unsaturated fatty acids. This composition is reported to serve for the prevention and treatment of atopic dermatitis, in addition to other purposes. More particularly, there has been disclosed a jelly which contains only 3 % of gamma-linolenic acid oil (corresponding to 8 % of gamma-linolenic acid in the oil), which is said to serve to prevent a "hangover".

DE-A-34 03 251 relates to a pharmaceutical dietetic composition for use in the treatment of atopic disorders wherein effective amounts of one or more metabolite(s) of linoleic acid and one or more metabolites of the alpha-linolenic acid are contained. Moreover, it has been disclosed that upon the administration of gamma-linolenic acid in the form of Evening Primrose oil the biochemical deficits in manifest atopy are in part compensated for, and clinical improvements are provided.

EP-A-0 292 403 relates to prostaglandin-lipid formulations which may be used for the treatment of, inter alia, bronchial asthma. An atopy prophylaxis has been mentioned nowhere.

EP-A-0 115 419 teaches the treatment of atopic diseases. It, therefore, is concerned with the already clinically manifested disease. There is no teaching for the prevention (prophylaxis) of atopic disease at a time without any manifestation of clinical disease. EP-A-0 115 419 still believes that atopy is an inherited tendency to develop one or more of a group of diseases of which eczema, asthma and allergic rhinitis are the major ones (see page page 3, lines 29 to 31).

EP-A-0 092 085 suggests the substitution or nutritional enrichment of foods with gamma-linolenic acid for the treatment or prevention of diverse metabolic disorders. As disorders are mentioned coagulation, skin diseases, endocrinologic disorders, myocardial disorders, hepatic disorders, joint diseases, neurological or mental disturbances.

There is absolutely no indication to use gamma-linolenic acid or its derivatives for the prevention of atopic or allergic diseases.

Patent Abstract of Japan, Vol. 12, No. 37 (C-473) [2884] describes the supplementation of foods for the prevention of unbalanced nutrition. One of those supplied products is gamma-linolenic acid. The application focuses on the blending of seasonings such as soy souce. There is not teaching that there is any linkage to an atopy-preventive effect of gamma-linolenic acid.

Brit. Journ. of Dermatology (1987) 117, 11-19 presents the data of a double blind clinical trial of oral Evening Primrose oil for the treatment of atopic eczema. All patients who have entered this study already suffered from atopic eczema. The patients receiving Primrose oil showed a significantly greater reduction in inflammation than those receiving placebo. There is not indication of the prevention of atopic diseases prior to the clinical manifestation of atopic diseases like atopic eczema.

The present invention is derived from a new understanding of atopy as so far not disclosed in prior art. It has been known that atopy patients suffer from a lack in gamma-linolenic acid and the metabolites thereof. This lack is supposed to be attributable to a defect in the delta-6-desaturase, which in atopy
patients is no longer capable of ensuring a sufficient reaction of linoleic acid to gamma-linolenic acid. On the ground of this recognition there were investigations in the state of prior art which showed that the administration of gamma-linolenic acid, e.g. in the form of Evening Primrose oil, makes up for the mentioned biochemical defect as well as leads to an alleviation of atopic symptoms. Thus, in summary prior art includes the teaching that the biological defect as well as the consequence thereof, namely the acute atopic state, can be treated by administering gamma-linolenic acid and its immediate metabolites dihomo-gamma-linolenic acid and arachidonic acid. In any event, this constitutes a treatment method which presumes that the biochemical defect as mentioned exists. In as much as prophylaxis of atopy is referred to at all, this certainly is not understood to mean a prevention within the meaning of healing the mentioned biochemical defect. Atopy prophylaxis within the meaning of prior art, finally, only is aimed to prevent the acute manifest forms of atopy, while a thoroughgoing prophylaxis cannot be ensured by prior art.

Thus, it is the object of the present invention to use substances for preparing agents for atopy prophylaxis at a time when conditioning and thorough maturing of the cellular immune system has not yet been completed. The term prophylaxis here is understood to mean a primary prevention which results in an elimination of factors established as being injurious to health before said factors become effective.

This object now can be attained in a surprisingly simple way by using substances for preparing agents containing at least one substance selected from the group consisting of gamma-linolenic acid, dihomo-gamma-linolenic acid, the physiologically compatible salts, esters, amides, phospholipids, glycolipids thereof, Prostaglandin E₁ (PGE₁), PGE₁ derivatives and PGE₁ analogs.

These substances should preferably be present in baby food and/or should be supplied to the baby therewith.

Gamma-linolenic acid occurs in the oil of the evening Primrose (Oenothera biennis) and in the seeds of black currants. Besides, there is a number of other natural sources of gamma-linolenic acid (e.g. Borago officinalis). Dihomo-gamma-linolenic acid is obtainable by the process according to EP-A-0 252 716 from cultures of Mortierella, among other sources.

The invention contemplates the recognition that a delta-6-desaturase deficiency or a supply deficient in linoleic acid metabolites is the crucial cause for a subsequently occurring clinical manifestation of atopy for other reasons. For example, in the case of a lack in delta-6-desaturase, babys are not capable of normally metabolizing linoleic acid. Important metabolites of linoleic acid include, among others, gamma-linolenic acid and dihomo-gamma-linolenic acid. In the organism, dihomo-gamma-linolenice acid is metabolized to also form Prostaglandin E₁, among other products. According to the recognitions by the present inventors, Prostaglandin E₁, is the essential mediator (stimulus), besides the thymic hormones such as, e.g., thymopoietin, thymulin and thymostimulin, for the normal conditioning and thorough maturing of the immune system of the newborn and young baby. Now, the defect in maturing and differentiation of the immune system in an atopic person result in an excessive un-controlled formation of immunoglobulin E in the manifest stage. Then, the immune system of the atopic is unable to control the IgE serum levels which are increase after a contact with allergen. According to the inventor's recognition, a prophylaxis of atopy should already start immediately after the birth, at a time when a clinical manifestation most regularly is not yet to be observed ant the conditioning and thorough maturing of the cellular immune system have not yet been completed.

Preferably the agent according to the present invention is baby food.

Baby foods within the meaning of the present invention are foodstuffs in powderized, pulpy, dried or liquid consistency. They include, among others, certified milk, evaporated milk, partially adapted and fully adapted finished food, high-protein low-fat finished food, milk-free finished food, hypoallergenic finished food and goat's milk preparations.

It is preferred to make agents wherein the substances are contained in liposomes which in turn have been formed from phospho- and/or glycolipids. Preferred are those liposomes formed from phospho- and/or glycolipids of the gamma-linolenic acid and/or dihomo-gamma-linolenic acid. A particularly preferred agent contains Prostaglandin E₁, its derivatives and/or analogs in the liposomes.

In a preferred embodiment of the process according to the invention, gamma-linolenic acid, dihomo-gamma-linolenic acid, the physiologically compatible salts, esters, amides, phospholipids, glycolipids thereof are provided in a concentration sufficient to ensure a daily dose of more than 150 mg of gamma-linolenic acid or dihomo-gamma-linolenic acid. It is preferred that a daily dose of 400 to 600 mg is ensured.

The agent may also contain linoleic acid. Furthermore, an antioxidant, preferably vitamin E, vitamin C and/or derivatives thereof, may be present. As a further additive, Prostaglandin E₂ can be present. In a further preferred embodiment of the invention, the agent according to the invention contains Ca²⁺, Zn²⁺ and/or Fe²⁺ ions.

It is preferred that 100 g of the agent contain
a) 150 to 600 mg of gamma-linolenic acid and/or dihomo-gamma-linolenic acid;
b) 0.15 to 0.5 mg of physiologically compatible zinc salt;
c) 5 to 10 mg of ascorbic acid; and
d) about 0.7 mg of α-tocopherol.

In a further preferred embodiment of the invention, the agent contains thymus hormones. These preferably are thymopoietin, thymulin, thymostimulin or thymopentin acetate.

In the process according to the invention, the substances are preferably dried, spray-drying or lyophilization being particularly preferred.

In a further preferred embodiment of the process according to the invention, the substances are dried together with albumin, and preferably hen's egg albumin.

A further subject matter of the present invention is the use of a thymus hormone for preparing agents for atopy prophylaxis by means of gamma-linolenic acid, dihomo-gamma-linolenic acid, the physiologically compatible salts, esters, amides, phospholipids, glycolipids thereof, and PGE₁, PGE₁ derivatives, PGE₁ analogs, wherein the thymus hormone is separately provided.

In the subject matters according to the invention it is preferred to provide a daily dose of thymus hormone of from 0.2 to 2 mg/kg of body weight.

The eminent advantage of the present invention is the prophylaxis of atopy, a disease which already now concerns more than 10 % of the people. This advantage is realized by that in a critical phase of the post partum maturing of the immune system a baby is supplied with sufficient amounts of those substances which, according to the new recognition by the present inventors are determining for forming a well-balanced functional immune system. More specifically, the administration at the same time or at different times of gamma-linolenic acid and/or its metabolites and the PGE₁ derivatives and/or analogs together with thymus hormones results in the desired effect. Thereby, in babies additionally supplied with the agent for atopy prophylaxis according to the present invention, regular thorough maturing of the immune system already in the baby age is ensured and a subsequent outbreak of manifest atopy forms is no longer to be feared.

## Claims

1. Use of gamma-linolenic acid, dihomo-gamma-linolenic acid, the physiologically compatible salts, esters, amides, phospholipids, glycolipids thereof, PGE₁, PGE₁ derivatives, and PGE₁ analogs for preparing agents for atopy prophylaxis at a time when conditioning and thorough maturing of the cellular immune system has not yet been completed.

2. The use according to claim 1, characterized in that the agent is baby food.

3. The use according to claim 1 or 2, characterized in that the agent additionally comprises a thymus hormone.

4. The use according to claim 3 wherein the thymus hormone is separately provide.

5. The use according to claim 3 or 4, characterized in that the agent contains a daily dose of thymus hormone of from 0.2 to 2 mg/kg of body weight.

## Patentansprüche

1. Verwendung von γ-Linolensäure, Dihomo-γ-linolensäure, der physiologisch verträglichen Salze, Ester, Amide, Phospholipide, Glycolipide davon, von PGE₁, PGE₁-Derivaten und PGE₁-Analoga zur Herstellung von Mitteln zur Atopieprophylaxe zu einer Zeit, wenn die Konditionierung und völlige Reifung des zellulären Immunsystems noch nicht beendet ist.

2. Verwendung gemäß Anspruch 1, dadurch gekennzeichnet, daß es sich bei dem Mittel um Babynahrung handelt.

3. Verwendung gemäß Anspruch 1 oder 2, dadurch gekennzeichnet, daß das Mittel zusätzlich ein Thymushormon umfaßt.

4. Verwendung gemäß Anspruch 3, wobei das Thymushormon getrennt bereitgestellt wird.

5. Verwendung gemäß Anspruch 3 oder 4, dadurch gekennzeichnet, daß das Mittel eine Tagesdosis an Thymushormon von 0,2 bis 2 mg/kg Körpergewicht enthält.

## Revendications

1. Utilisation de l'acide γ-linolénique, de l'acide homo-γ-linolénique, des sels, esters, amides, phospholipides, glycolipides physiologiquement compatibles de ceux-ci, de PGE₁, des dérivés de PGE₁ et des analoques de PGE₁ pour la préparation de produits prophylactiques pour l'atopie qui sont mises en oeuvre à un moment où le conditionnement et la maturation entière du système d'immunité cellulaire n'ont pas encore été complétés.

2. Utilisation selon la revendication 1, caractérisée en ce que ledit produit est de la nourriture pour bébés.

3. Utilisation selon l'une des revendications 1 ou 2, caractérisée en ce que ledit produit comprend de plus une hormone thymique.

4. Utilisation selon la revendication 3, où ladite hormone thymique est disposée séparément.

5. Utilisation selon l'une des revendications 3 ou 4, caractérisée en ce que ledit produit contient une dose quotidienne d'hormone thymique qui est comprise entre 0,2 et 2 mg/kg de poids du corps.
